# EUROPEAN PATENT APPLICATION

(11) **EP 4 574 138 A1**
(43) Date of publication of application: **25.06.2025**
(21) Application number: 24221332.0
(22) Date of filing: 19.12.2024
(51) Int. Cl.: A61K 9/20, A61K 31/47

(54) **A TABLET COMPOSITION COMPRISING IVACAFTOR**

(30) Priority: 20.12.2023 TR 202317799; 15.05.2024 TR 202405912
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); GULER, Tolga, Istanbul (TR); KIR, Ufuk, Istanbul (TR); GOKSEN, Elif, Istanbul (TR)
(74) Representative: Genç Ilhan, Oznur

(57) **Abstract**

The present invention relates to a tablet composition comprising an intragranular phase and an extragranular phase, wherein the intragranular phase comprising ivacaftor and the extragranular phase comprising at least two surfactants. The present invention also relates to a simple, rapid, cost-effective, time-saving and industrially suitable process for the preparation of a tablet composition.

## Description

### Field of the Invention

The present invention relates to a tablet composition comprising an intragranular phase and an extragranular phase, wherein the intragranular phase comprising ivacaftor and the extragranular phase comprising at least two surfactants. The present invention also relates to a simple, rapid, cost-effective, time-saving and industrially suitable process for the preparation of a tablet composition.

### Background of the Invention

Ivacaftor, also known as N-(2,4-di-tert-butyl-5-hydroxyphenyl)-1,4-dihydro-4-oxoquinoline-3-carboxamide, having the following Formula (I):

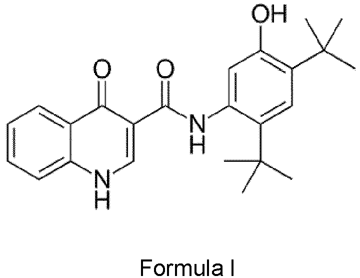

Ivacaftor is a white to off-white powder that is practically insoluble in water (<0.05 microgram/mL). Ivacaftor has low water solubility, very lipophilic and bioavailability of Ivacaftor is significantly enhanced when co-administered with food. Due to poor aqueous solubility, extensive formulation efforts were required and resulted in a spray-dried dispersion of ivacaftor suitable for oral administration. Ivacaftor was approved by FDA and marketed by vertex pharma for the treatment of cystic fibrosis under the brand name KALYDECO^{®} in the form of 150 mg oral tablets. Kalydeco^{®} is indicated for the treatment of cystic fibrosis in patients age 6 years and older who have a G55ID mutation in the CFTR (cystic fibrosis transmembrane conductance regulator) gene.

Ivacaftor is a potentiator of the CFTR protein. The CFTR protein is a chloride channel present at the surface of epithelial cells in multiple organs. Ivacaftor facilitates increased chloride transport by potentiating the channel-open probability (or gating) of the CFTR protein.

Accordingly, there is a need for stable, bioavailable and high solubility pharmaceutical compositions of Ivacaftor useful for treating patients suffering from cystic fibrosis.

The patent WO2022013360A1 disclose an immediate release tablet composition comprising free particles of ivacaftor a pH dependent polymer and surfactant extragranularly and intragranularly.

The patent WO2010019239A2 disclose a pharmaceutical composition comprising a solid dispersion of ivacaftor, methods of manufacturing pharmaceutical compositions of the composition.

It is known in the prior art that the solubility of ivacaftor is poor and there are patent applications written regarding this. Prior to this, there are studies on increasing the dissolution profile and stability. In one example, stability was increased by using a pH dependent polymer and a surfactant. In another example, the desired dissolution profile was achieved with the active ingredient ratio. There is still a need for a simple formulation that simultaneously solves both the stability and ivacaftor dissolution problem.

### Detailed Description of the Invention

The main object of the present invention is a tablet composition comprising ivacaftor having the desired dissolution profile and stability.

Another object of the present invention is a tablet composition comprising ivacaftor having content uniformity, flowability and compressibility.

Another object of the present invention is to provide a preparation process of the tablet composition comprising ivacaftor which is simple and cost-effective process.

The term "Ivacaftor" as used herein refers to Ivacaftor in the form of free base or in the form of pharmaceutically acceptable salts, crystalline polymorph, solvates, hydrates, esters or in amorphous form or mixture thereof.

Surfactants are widely used to enhance the dissolution of various water-insoluble drugs. It is known in the prior art that ivacaftor is insoluble in water. A new formulation is still needed for this. We found that the use of at least two surfactants in the extragranular phase increased the solubility. In this way, we found that while providing the desired dissolution profile, high stability was also achieved at the same time.

According to one embodiment of the present invention, the tablet composition comprises;
a) an intragranular phase comprising ivacaftor,
b) an extragranular phase,
wherein the extragranular phase comprising at least two surfactants.

According to one embodiment of the invention, the extragranular phase comprises surfactants, disintegrant, glidant and lubricant.

Suitable surfactants are selected from the group comprising sodium lauryl sulfate, poloxamer P338, cetostearyl alcohol, cetomacrogol emulsifying wax, gelatin, casein, docusate sodium, benzalkonium chloride, calcium stearate, polyethylene glycols, phosphates, polyoxyethylene sorbitan fatty acid esters, tragacanth, polyoxyethylene 20 stearyl ethers, polyoxyethylene alkyl ethers, polyoxyethylene castor oil derivatives, pegylated hydrogenated castor oils, sorbitan esters of fatty acids, Vitamin E or tocopherol derivatives, vitamin E TPGS, tocopheryl esters, lecithin, phospholipids and their derivatives, stearic acid, oleic acid, oleic alcohol, cetyl alcohol, mono and diglycerides, propylene glycol esters of fatty acids, ethylene glycol palmitostearate, polyoxylglycerides, propylene glycol monocaprylate, propylene glycol monolaurate, alkyl aryl polyether alcohols and polyglyceryl oleate or a mixture thereof.

According to an embodiment of the present invention, sodium lauryl sulfate and poloxamer P338 are used as surfactants in the extragranular phase.The combined use of these two surfactants increased the solubility and stability.

According to this embodiment of the present invention, the amount of surfactant is between 0.01% and 10.0% by weight of the total formulation. Preferably, the amount of surfactant is between 1.0% and 8.0% by weight of the total formulation.

Suitable disintegrants are selected from the group comprising croscarmellose sodium, calcium carboxymethyl cellulose, crospovidone, starch, sodium carboxymethyl cellulose, carboxymethyl cellulose, docusate sodium, guar gum, polyacryline potassium, sodium alginate, sodium starch glycolate, alginic acid, alginates, ion-exchange resins, magnesium aluminium silica, sodium dodesyl sulphate, poloxamer, sodium glycine carbonate or mixtures thereof.

According to this embodiment of the present invention, croscarmellose sodium is used as disintegrant in the extragranular phase.

According to this embodiment of the present invention, the amount of disintegrant in the extragranular phase is between 0.1% and 8.0% by weight of the total formulation. Preferably, the amount of disintegrant in the extragranular phase is between 1.0% and 5.0% by weight of the total formulation.

Suitable lubricants are selected from the group comprising magnesium stearate, fatty acid, stearic acid, calcium stearate, sodium stearate, stearic acid, glyceryl monostearate, hydrogenated oils (i.e. hydrogenated vegetable oil), polyethylene glycol, fatty alcohol, fatty acid ester, glyceryl behenate, mineral oil, vegetable oil, leucine, sodium benzoate, or a mixture thereof.

According to an embodiment of the present invention, magnesium stearate is used as lubricant in the extragranular phase.

According to this embodiment of the present invention, the amount of lubricant is between 0.01% and 5.0% by weight of the total formulation.

Suitable glidants are selected from the group comprising colloidal anhydrous silica, magnesium oxide, magnesium silicate, hydrophobic colloidal silica, calcium phosphate, powdered cellulose or a mixture thereof.

According to an embodiment of the present invention, colloidal anhydrous silica is used as glidant in the extragranular phase.

According to this embodiment of the present invention, the amount of glidant is between 0.01% and 5.0% by weight of the total formulation.

According to one embodiment of the invention, the intragranular phase comprises ivacaftor, diluent, binder and disintegrant.

According to one embodiment of the invention, the amount of ivacaftor is between 15.0% and 35.0% by weight of the total formulation. Preferably, the amount of ivacaftor is between 20.0% and 30.0% by weight of the total formulation.

Suitable diluents are selected from the group comprising lactose monohydrate, lactose, dibasic calcium phosphate, mannitol, spray-dried mannitol, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or a mixture thereof.

According to an embodiment of the present invention, lactose monohydrate is used as the diluent in the intragranular phase.

According to this embodiment of the present invention, the amount of diluent is between 20.0% and 40.0% by weight of the total formulation. Preferably, the amount of diluent is between 25.0% and 35.0% by weight of the total formulation.

Suitable binders are selected from the group comprising microcrystalline cellulose, polyvinylprolidone-vinyl acetate copolymer (PVP-VA), corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or a mixture thereof.

According to this embodiment of the present invention, microcrystalline cellulose is used as the binder in the intragranular phase.

According to this embodiment of the present invention, the amount of binder in the intragranular phase is between 15.0% and 35.0% by weight of the total formulation. Preferably, the amount of binder in the intragranular phase is between 18.0% and 28.0% by weight of the total formulation.

According to this embodiment of the present invention, used disintegrant in the intragranular phase is selected from the disintegrant group the described above. Croscarmellose sodium is used as disintegrant in the intragranular phase.

According to this embodiment of the present invention, the amount of disintegrant in the intragranular phase is between 1.0% and 10.0% by weight of the total formulation. Preferably, the amount of disintegrant in the intragranular phase is between 3.0% and 8.0% by weight of the total formulation.

The disintegrant is used both in the intragranular phase and in the extragranular phase. The use of the disintegrant in both stages provided the desired content uniformity while also increasing the dissolution profile.

As used here in, `particle size' means the cumulative volume size distrubition as tested by any conventionally accepted method such as the laser diffraction method (i.e. malvern analysis). The term d (0.9) means, the size at which 90% by volume of the particles are finer. The term d (0.5) means, the size at which 50% by volume of the particles are finer.

According to this embodiment of the present invention, ivacaftor in the form of the free base or in the form of pharmaceutically acceptable salts has a d (0.9) particle size between 37 µm and 70 µm.

According to this embodiment of the present invention, ivacaftor in the form of the free base or in the form of pharmaceutically acceptable salts has a d (0.9) particle size between 46 µm and 63 µm, preferably between 48 µm and 58 µm. The use of particle size at these ratios helped provide dissolution profile.

In the present invention, the tablet composition comprises film coating to protect the composition against the moisture and light to maintain the stability. Suitable film coating agents are selected from the group comprising polyvinyl alcohol (PVA), titanium dioxide, macrogol (PEG 3350), Indigo carmine aluminium lake, talc, carnauba wax, hydroxypropylmethylcellulose, polyethylene glycol (PEG), polyvinyl alcohol-polyethylene glycol copolymers, lecithin, pigments, dyes, iron oxide or a mixture thereof.

According to this embodiment of the present invention, the amount of film coating agent is between 1.0% and 5.0% by weight of the total formulation.

Wet granulation is used for the intragranular phase. It also comprises binder solution for wet granulation. The binder solution comprises at least one binder and a solvent. The binder is selected from the binder group described above. Preferably copovidone is used as binder in the binder solution.

Suitable solvents are selected from the group comprising pure water, propylene glycol, glycerine, alcohols, polyethylene glycol, ethanol, isopropyl alcohol or their mixtures thereof.

According to an embodiment of the present invention, the solvents is pure water.

According to this embodiment of the present invention, the tablet composition comprises;
a) the intragranular phase comprising ivacaftor,
b) the binder solution comprising a binder and a solvent,
c) the extragranular phase comprising Poloxamer and SLS as surfactants.

According to one embodiment of the present invention, the tablet composition comprising ivacaftor or a pharmaceutically acceptable salt thereof is obtained by wet granulation process. This method provides the desired compressibility and flowability without loss of active substance.

A process for preparing a tablet composition comprising ivacaftor comprises the following steps;
a. weighing ivacaftor and all excipients,
b. mixing the intragranular phase,
c. sieving the mixture through sieve,
d. then, performing a binder solution with a binder and a solvent,
e. mixing the mixture at step (c) and the binder solution,
f. drying the mixture in step (e) and sieving,
g. sieving each of the extragranular phase, adding them to the mixture in step (f) and mixing,
h. compressing the resulting homogeneous mixture in accordance with the specifications specified for each tablet,
i. coating the tablets with film coating.

### Example 1;

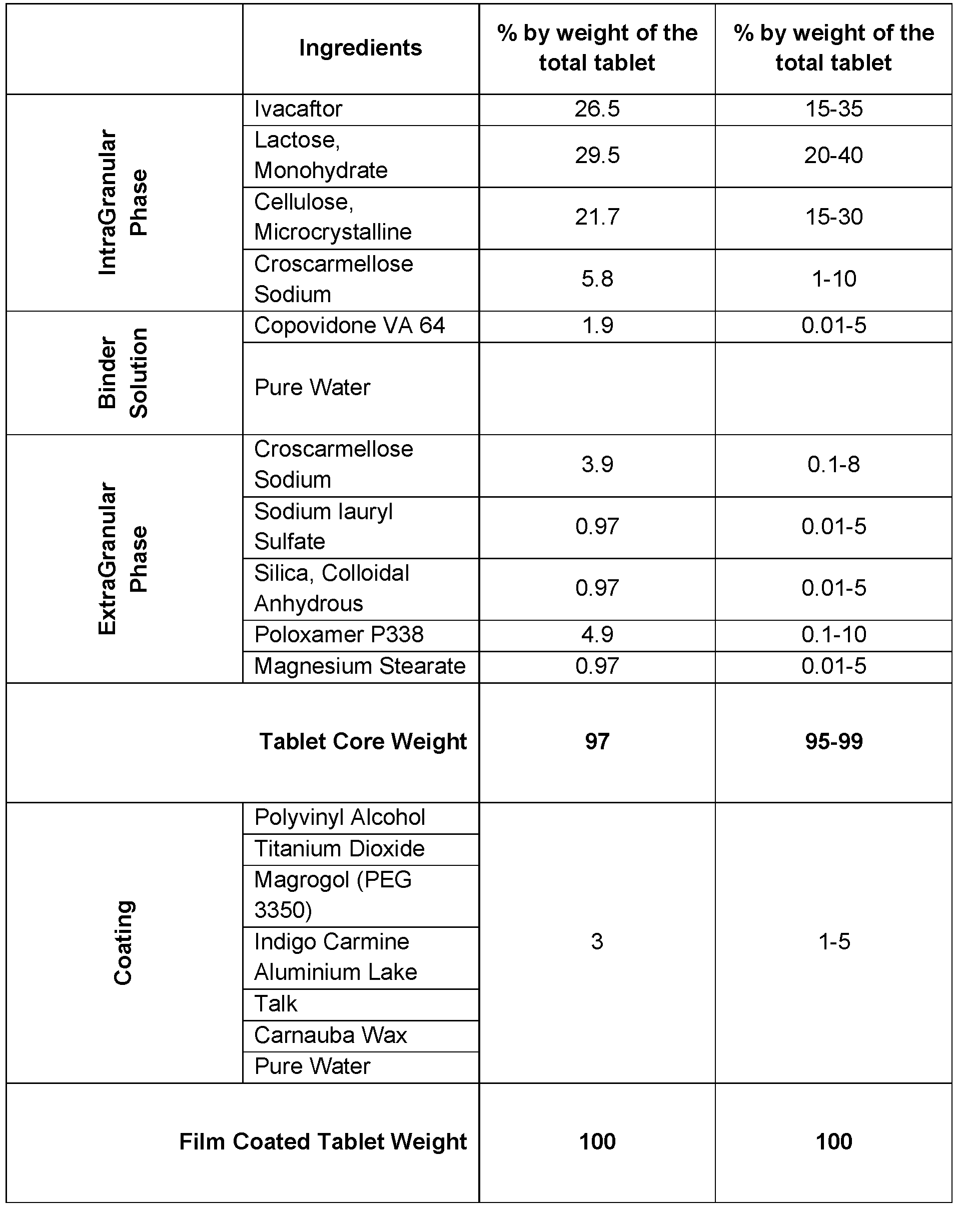

a) weighing ivacaftor and all excipients,
b) mixing lactose monohydrate, microcystralline cellulose, croscarmellose sodium and ivacaftor and obtained intragranular phase,
c) sieving the mixture through sieve,
d) dissolving copovidone in pure water and obtained a binder solution,
e) then, granulating the binder solution and the mixture in step (c),
f) drying the mixture in step (e) and sieving,
g) sieving each of the croscarmellose sodium, sodium lauryl sulfate, colloidal anhydrous silica, poloxamer p338, magnesium stearate, adding them to the mixture in step (f) and mixing,
h) compressing the resulting homogeneous mixture in accordance with the specifications specified for each tablet,
i) coating the tablets with film coating.

## Claims

1. A tablet composition comprises;
d) the intragranular phase comprising ivacaftor,
e) the extragranular phase,
wherein the extragranular phase comprising at least two surfactants.

2. The tablet composition according to claim 1, wherein the extragranular phase comprises surfactants, disintegrant, glidant and lubricant.

3. The tablet composition according to claim 1 or 2, wherein surfactants are selected from the group comprising sodium lauryl sulfate, poloxamer P338, cetostearyl alcohol, cetomacrogol emulsifying wax, gelatin, casein, docusate sodium, benzalkonium chloride, calcium stearate, polyethylene glycols, phosphates, polyoxyethylene sorbitan fatty acid esters, tragacanth, polyoxyethylene 20 stearyl ethers, polyoxyethylene alkyl ethers, polyoxyethylene castor oil derivatives, pegylated hydrogenated castor oils, sorbitan esters of fatty acids, Vitamin E or tocopherol derivatives, vitamin E TPGS, tocopheryl esters, lecithin, phospholipids and their derivatives, stearic acid, oleic acid, oleic alcohol, cetyl alcohol, mono and diglycerides, propylene glycol esters of fatty acids, ethylene glycol palmitostearate, polyoxylglycerides, propylene glycol monocaprylate, propylene glycol monolaurate, alkyl aryl polyether alcohols and polyglyceryl oleate or a mixture thereof.

4. The tablet composition according to claim 3, wherein sodium lauryl sulfate and poloxamer P338 are used as surfactants in the extragranular phase.

5. The tablet composition according to claim 3 or 4, wherein the amount of surfactant is between 0.01% and 10.0% by weight of the total formulation.

6. The tablet composition according to claim 2, wherein croscarmellose sodium is used as disintegrant in the extragranular phase.

7. The tablet composition according to claim 6, wherein the amount of disintegrant in the extragranular phase is between 0.1% and 8.0% by weight of the total formulation.

8. The tablet composition according to claim 1, wherein the intragranular phase comprises ivacaftor, diluent, binder and disintegrant.

9. The tablet composition according to claim 1, wherein the amount of ivacaftor is between 15.0% and 35.0% by weight of the total formulation.

10. The tablet composition according to claim 8, wherein microcrystalline cellulose is used as the binder in the intragranular phase.

11. The tablet composition according to claim 8, wherein croscarmellose sodium is used as disintegrant in the intragranular phase.

12. The tablet composition according to claim 11, wherein the amount of disintegrant in the intragranular phase is between 1.0% and 10.0% by weight of the total formulation.

13. The pharmaceutical combination according to any preceding claim, wherein further comprising the binder solution which comprises at least one binder and a solvent.
